# EUROPEAN PATENT SPECIFICATION

(11) **EP 1 661 512 B1**
(45) Date of publication and mention of the grant of the patent: **21.04.2010**
(21) Application number: 04028047.1
(22) Date of filing: 25.11.2004
(51) Int. Cl.: A61B 5/0408, A41D 13/12

(54) **Portable wireless electrocardiogram monitoring device**
Drahtlos arbeitendes, tragbares elektrocardiographisches Überwachungsgerät
Appareil portable sans fil de surveillance d'électrocardiogrammes

(43) Date of publication of application: 31.05.2006
(73) Proprietor: Taiwan Textile Research Institute, Tucheng City, Taipei Country 236, Taiwan (TW)
(72) Inventor: Shen, Chien-Lung, Chiayi City (TW); Li, Chun-Hui, Banciao City Taipei County (TW); Kun-Chi, Hsieh, Sanmin District Kaohsiung City (TW); Cheng, Yen-Chun, Hsilo Township Yunlin County (TW)
(74) Representative: Kador & Partner

(56) References cited:
- EP-A- 0 966 917
- WO-A-03/094717
- DE-A1- 10 056 072
- US-A1- 2003 212 319
- US-A1- 2004 138 546

## Description

### BACKGROUND OF THE INVENTION

The present invention relates to a wireless transmitted electrocardiogram monitoring device which uses conductive fabric as electrodes in combination with wireless transmission for monitoring human electrocardiogram.

Refer to European Patent No. 0375440A1 -"An electrode for use on living tissue", filed on 1990, an electrode for use in electrocardiography is disclosed. The device comprises a moisture vapour permeable porous material and an electrically conductive powder. The electrode may have an adhesive on one surface to facilitate attachment to a patient. As to another World Intellectual Property Organization patent No. WO-03094717A1-"TEXTILE ARTICLE HAVING ELECTRICALLY CONDUCTIVE PORTIONS AND METHOD FOR PRODUCING THE SAME", filed in 2003, a textile garment in the form of a bra or ladies top produced by circular knitting technique for detecting electrocardiogram is disclosed. The knitted construction includes electrically conductive yarn (fiber coated with metal) to form electrodes. The electrode is arranged in the inner layer, while the outer layer is an electrically insulated layer.

Moreover, in a Japanese patent publication No. 2000000221A2 named "SHEET FOR ELECTRODE", electrodes disposed inside a sheet for detecting electrocardiogram waveform and heart beat rate is disclosed. The electrodes are arranged near user's head and feet. The weft of the fabric is conductive yarn while the warp is non-conductive yarn. As to another Japanese Patent No. 60079957A2-"ENERGIZATION HEAD", applied on 1985, a fabric electrode made by fiber with diameter of 70 µm that is made of stainless filaments with diameter of 30 µm intertwisted with polyester (PET) fiber with diameter of 20 µm. The mixture of epoxy resin and aluminum hydroxide is heated to coat the fabric electrode. Refer to a further Japanese Patent No.06070897A2-"ELECTRODES MOUNTING STRUCTURE IN VEST FOR ELECTROCARDIOGRAM MEASUREMENT", a vest for long-term detection of electrocardiogram is disclosed. The positions for measuring are around the front side of the chest and the belly. The vest is washable and with a slot for installation and disassembling of the external electrodes.

Moreover, refer to US patent No. 4,608,987-"Apparatus for transmitting ECG data", A vest-like garment is provided. The device includes a front fabric panel with a plurality of apertures adapted for receiving associated electrodes, a horizontally stretchable rear panel, and a pair of vertically stretchable shoulder straps. Each of the electrodes is biased against the front panel for better electrical engagement with the skin of the user. Leads from each electrode may carry signals to a telephonic transmission unit, which is also equipped with emergency electrode handles. As to the US patent No. 4,026,278/US4,729,37 named "Electrode positioning and retaining belt", an electrode positioning belt for electrocardiogram is disclosed. An electrode positioning belt has Velcro loop fabric along the inner surface. Velcro hook fabric may be affixed to the back of the electrodes so they may be removable secured to the belt. The belt is wrapped around a body member. The ends of the belt overlap and a tab of hook fabric at one end of the belt on the outer surface is used to secure the belt. US patent No. 4,580,572 -"Garment apparatus for delivering or receiving electric impulses" disclosed a garment containing multiple conductive paths made of conductive cloth is used to connect an external electrical apparatus to various points on the skin of the wearer. The garment can be designed for electrical monitoring of physical status. A fabric, in the form of a woven or knitted fabric or garment disclosed in US patent No. 6,381,482-"Fabric or garment with integrated flexible information infrastructure" is used for collecting, processing, transmitting and receiving information. The fabric allows a new way for information processing by selecting and plugging in (or removing) components from the fabric. The fabric can also be provided with sensors for monitoring physical aspects of the wearer. US patent No. 6,145,551-"Full-fashioned weaving process for production of a woven garment with intelligence capability" discloses a woven garment made of only one single integrated fabric with armholes includes intelligence capability to monitor one or more body vital signs by including a selected sensing component or components.

Refer to Taiwanese patent publication No. 150548, a monitoring device for human heart beat, blood pressure, respiration and body temperature is disclosed. The monitoring device has a screen for displaying, voice and video signals for alarm and electrodes for detecting and amplifying weak signals from user's chest. As to Taiwanese patent publication No. 333445, a long-term monitoring method for cardiac activities is disclosed. The method includes pre-processing, reducing noise, and measuring threshold value of heart beating signals or noise in a dynamic way. Then the electrocardiogram waveforms are classified according to respective features of waves. Refer to Taiwanese patent publication No. 286538, it relates to a monitoring system for people in motion. The device, having a portable power supply and a microprocessor, measures and stores electrocardiogram and blood pressure data for diagnosis reference. A wireless measuring device for physical parameters is disclosed in Taiwanese patent publication No. 243179. The device includes a detector for body temperature and pulse, connected to a conversion circuitry for converting detected signals into physical parameters such as body temperature and pulse. Taiwanese patent publication No. 241002 named earphone-type detector for physical status discloses a detector composed by a detecting unit, in combination with a detecting module and a signal conversion module for receiving signals of physical status, and a beeper with warning voices for reminding users that the detected value is higher than normal range. Taiwanese patent publication No. 567831 discloses a bluetooth body temperature monitor consists of a bluetooth host and a bluetooth detect for body temperature detection. Taiwanese patent publication No. 526649 discloses a personal health monitor system using mobile phone for monitoring user's physical status. The users' data of physical status is collected and processed by monitoring program inside a portable information processor, then a corresponding message is produced to inform user about his/her health situation. Taiwanese patent publication No. 454502 discloses a monitor for detecting physical status with function of mobile phone. A mobile phone receiving physical signals displays values of signal on the phone screen in dot matrix way. Taiwanese patent publication No. 448761 discloses an underwear for monitoring heart beat arranged on chest. A contact point is attached on user's chest for detecting pulse from heart beats and then transmitted to the host in wireless way. Taiwanese patent publication No. 366796 discloses a wireless transmitter with infrared sensor for heat beat composed by a circuitry for reducing noise, heart heat sensor, and an amplifier transmitter. Taiwanese patent publication No. 275790 discloses a conductive piece attached on front side of the chest. By detecting electrical activity of heart form conductive piece placed on the surface of the skin, the detected signal is amplified and filtered through circuitry, then estimate heart rate by a heart beat detector. In Taiwanese patent No. M243180, strap structure for heat beat detector is disclosed. Two sheets of electrodes penetrate through air-permeable fabric and fixed on circuitry by metal rivets to form a strap tied on chest or other positions of body.

Then the heart beat is estimated according to potential difference of pulse measured by the electrode contacted with the skin.

EP 0966917 describes a screen for protecting a medical measuring device against external disturbances e.g. static electricity. The screen is made of a plastic-like supporting material.

However, Taiwanese patents No. 150548, 333445, 286538, 243179, 241002, 567831, 526649, 454502, 448761, 366796, 275790, and M243180 has following shortcomings:
1. Most detectors of detecting devices or systems for physical status disclosed in above patents are portable or wearable for attaching on clothes or easy carrying. They are somewhat inconvenient for use. 2. The detectors of the above devices are made by hard material. Thus when they contact the skin of users, they can't adsorb moisture and eject sweat and they may cause problems of skin such as allergy. 3. The design of detectors on above sensing devices or systems has no softness and comfort so that they are not suitable for long-term or nonstop monitoring because they may cause skin discomfort. 4. The appearance of above monitoring devices or systems is easily be seen or attended and the devices are not minimized. Thus there must be some space for accommodating the devices or held by users. Thus the wearing of the devices causes some interference for users and can't be used without being feeling of devices.

Furthermore, the disadvantages of US patent No. 4608987, US patent No. 4580572, European patent No. 375440A1, Japanese patent No. 6070897A2, and Japanese patent No. 60079957A2 are: (1)The detecting component in the soft goods of above patents is made of cold and hard metal. When the detecting component touches user's skin, it may cause uncomfortable feelings. (2) The detector and the controller are connected by external wires (nonconductive fiber) so that the storage and breakage of these external wirings cause inconvenience of users. (3) The electrodes or detecting component composed by fibered porous material is not popular on the market. And the features (such as strength, elasticity, and absorption) of the material are far more less than the yarn. (4) The fabric electrodes on above soft goods are processed and hardened. When being applied to a polarized electrode, it is durable and thus is not suitable for long-term use. (5) The above soft goods with detection function need to be assembled with external electrodes through complicated steps for detection. Thus they can't be applied frequently on daily lives.

In addition, the shortcomings of the Japanese patent publication No. 2000000221A2, US patent No. 6381482, US patent No. 6145551 and WO patent No. 3094717A1 are :
(1)the soft goods mentioned in above patents are not portable or without the convenience of wear. (2) The detectors inside above soft goods need to combine with large area of fabric for achieving substantial effect.

Thus there is a need to provide a device having electrodes with features of comfort, air permeability, softness, and stretchability and signals being detected are transmitted in wireless way so as to avoid feelings of being tied and suitable for long-term monitoring of users' physical status.

### SUMMARY OF THE INVENTION

Therefore it is a primary object of the present invention to provide a wireless transmitted electrocardiogram monitoring device which includes electrodes made of conductive fabric with features of comfort, air permeability, softness, and stretchability as well as wireless transmission for long-term monitoring of users' physical signals.

It is another object of the present invention to provide a wireless transmitted electrocardiogram monitoring device which uses at least three electrodes made of conductive fabric, wherein two electrodes are disposed on the fourth rib counting backwards of right and left chest respectively and one electrode is arranged on back of a human body. The three electrodes made of conductive fabric are connected with textile by weaving so as to avoid feelings of being bound.

It is a further object of the present invention to provide a wireless transmitted electrocardiogram monitoring device that transmits monitoring data to a portable device such as PDA (personal digital assistant) in wireless way. Because the size of the portable device can be minimized with quite small volume so that it's convenient to be arranged -for example by handhold or in the pocket and is suitable for long-term monitoring.

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein
Fig. 1 is a block diagram of a preferred embodiment in accordance with the present invention;
Fig. 2A is a schematic diagram showing the front position of electrodes made of conductive fabric being disposed in accordance with the present invention;
Fig. 2B is a schematic diagram showing the rear position of electrodes made of conductive fabric being disposed in accordance with the present invention;
Fig. 3 is a schematic diagram showing the structure of fabric in accordance with the present invention to protect users from electromagnetic waves.

### DETAILED DESCRIPTION OF THE PREFFERED EMBODIMENT

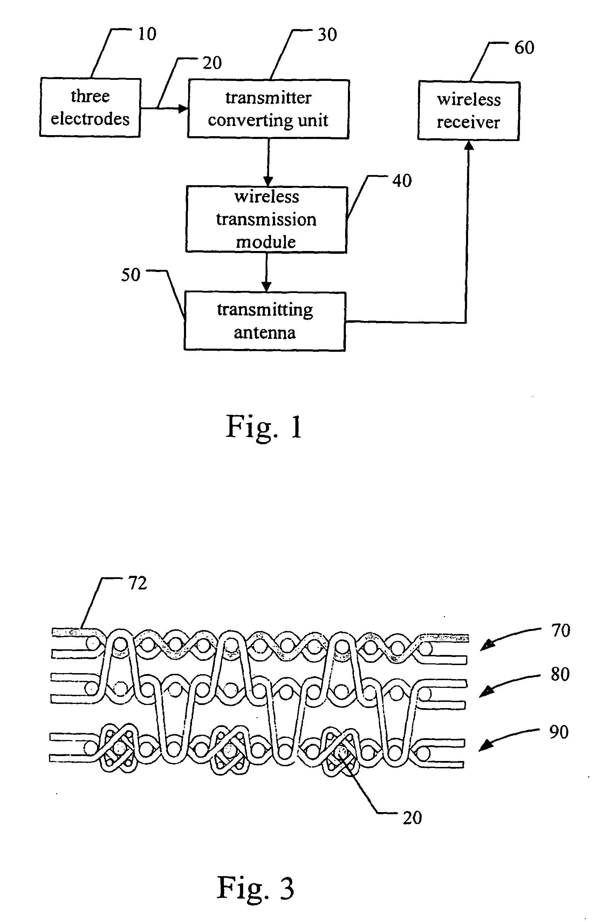

Refer to Fig.1, a preferred embodiment of the present invention is disclosed. A wireless transmitted electrocardiogram monitoring device in accordance with the present invention consists of at least three electrodes made of conductive fabric 10 having conductive fiber and nonconductive fiber, a transmitter converting unit 30 connected to the three electrodes 10 by at least three conductive yarn 20; the transmitter converting unit 30 receives an input signal and converts it into a transmitted signal; a wireless transmission module 40 for receiving the transmitted signal and producing a corresponding wireless signal; and a transmitting antenna 50 receiving the signal and transmitting it into a wireless receiver 60.

The features of the electrodes made of conductive fabric are described as followings:
Processed by plain weave or knitting with surface resistance: 0.16 Ω/cm²; specification of fiber:
   - conductive fiber (A): yarn coated with silver 150D/72f
   - nonconductive fiber (B): PET 150D/48f
   - processing conditions: 2(A)1(B) 214 TPM

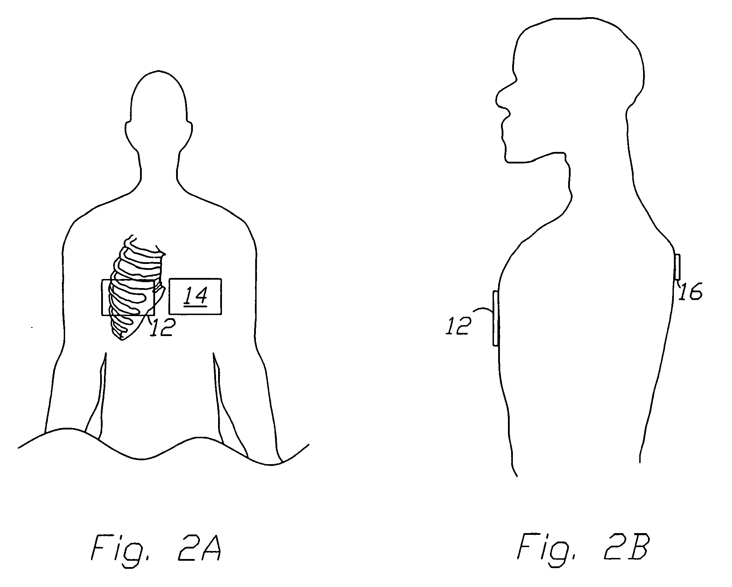

Refer to Fig. 2A & Fig. 2B, a schematic drawing shows positions of the electrodes made of conductive fabric in accordance with the present invention. In the three electrodes made of conductive fabric 10, two electrodes made of conductive fabric 12, 14 are disposed near the fourth rib counting backwards of right and left chest of the tights respectively while one electrode 16 is arranged on rear side of an underwear, close to the skin.

Refer to Fig.3, a preferred embodiment of the present invention that protects users from electromagnetic waves is disclosed. As shown in figure, outside the electrode made of conductive fabric 10 and the conductive yarn 20, there are an electromagnetic waves shielding layer 70, an isolation layer 80, and a conducive wire fixing layer 90 from outside in. The electromagnetic interference shield layer 70 is a shield for preventing electromagnetic waves woven by conductive yarn 72 while the isolation layer 80 is woven by nonconductive yarn. And the conducive wire fixing layer 90 is made by laminated fabric that is double layers structure for fixing and securing the conductive yarn 20 or the conductive yarn of the electrode 10. The composition and weaving conditions are as following:
1.Wales Per Inch : 180 (the number of wales)/inch
2.Course Per Inch : 180 (the number of courses)/inch
3.warp : A= pet 150 d **B** = the silver fiber
4.weft : A= pet 150 d **B** = the silver fiber
5.arrangement of warp : A108^{,}(1B2A)*88^{,}A108^{,}(1B2A)*70^{,}A108^{,} (1B2 A)*88 ^{,} A108
6.harness draft : (1.2.3.4.5.6.1.7.3.4.8.6)* 24 ^{,} [(1.9.3.4.10.6.1.11.3.4.12.6.) *11 ^{,} (1.9.3.4.10.6.1.13.1.415.16.3.4.13.14.15.16.6.17) ^{,} (1.13.1.415.16.3.4.13.14.15.16.6.1.11.3.4.12.6) ^{,} (1.9.3.4.10.6.1.11.3.4.12.6.)*11 (1.2.3.4.5.6.1.7.3.4.8.6)* 2] *3 ^{,} (1.2.3.4.5.6.1.7.3.4.8.6)* 22
7 arrangement of weft : 1B2 A

When a mobile phone in communication is close to human body, it interferes electrocardiogram signals, especially gain in the frequency of 100Hz and 120Hz, the electrocardiogram in spectrum is seriously affected. During the measurement process, except the user, if other people contact or approach the transmission pathway of the electrocardiogram signals, the interference of 60Hz from environment will be introduced into the signals. However, no matter the interference either from contacts or electromagnetic waves, the quality of signals is dramatically improved after using products of the present invention. quantization results

| | AC interference | Electromagnetic interference | |
|---|---|---|---|
| | **gain at 60Hz (unit)** | **gain at 100Hz (unit)** | **gain at 120Hz (unit)** |
| **Standard transmission line"** | **0.4729** | **10.3239** | **1.966** |
| **Product of embodiment according to the present invention** | **0.1233** | **0.0639** | **0.0273** |

### Embodiment 1: electrocardiogram monitoring strap

A. electrocardiogram detective unit
   1. Three electrodes made of conductive fabric are sewed on a chest belt respectively;
   2. Three conductive buttons are strung on three pieces of conductive fabric respectively for providing conductive pathway of electrodes made of conductive fabric and the electrocardiogram monitoring device with bluetooth interface;
   3. The end product is worn on the chest and signals being detected are transmitted to a PDA through the bluetooth interface.
B. The PDA is fixed on a watchband so that it can be carried on limbs of users.

### Embodiment 2: electrocardiogram monitoring costume

A. electrocardiogram detective unit
   1. In the three electrodes made of conductive fabric, two pieces of conductive fabric are sewed at the position near the fourth rib counting backwards of right and left chest respectively while the third piece of conductive fabric is arranged on back of an underwear, close to the skin.
   2. Three conductive buttons are strung on three pieces of conductive fabric respectively through the integrated four plies of conductive yarn for providing conductive pathway of electrodes made of conductive fabric and the electrocardiogram monitoring device with bluetooth interface.
   3. The conducive yarn has no contact with user's skin, only the fabric or yarn contacts the skin. The three conductive buttons also don't touch the skin, but through the fabric or isolation material.
   4. The end product is worn on the upper body and the detected signals are transmitted to the PDA through the bluetooth interface.
B. A pocket on sports coat is used as a carrier of a PDA for being carried by users conveniently.

### Embodiment 3: electrocardiogram monitoring waist belt

A. electrocardiogram detective unit
   1. In the three electrodes made of conductive fabric, two pieces of conductive fabric are sewed at the position near the fourth rib counting backwards of right and left chest respectively while the third piece of conductive fabric is arranged on back of an underwear, close to the skin.
   2. Three conductive buttons are strung on three pieces of conductive fabric respectively through the integrated four plies of conductive yarn for providing conductive pathway of electrodes made of conductive fabric and the electrocardiogram monitoring device with bluetooth interface.
   3. The conducive yarn has no contact with user's skin, only the fabric or yarn contacts the skin. The three conductive buttons also don't touch the skin, but through the fabric or isolation material.
   4. The end product is worn on the upper body and signals being detected are transmitted to a PDA through the bluetooth interface.
B. A waist belt is used as a carrier of a PDA for being carried by users easily.

Additional advantages and modifications will readily occur to those skilled in the art.

## Claims

1. A wireless transmitted electrocardiogram monitoring device comprising:
a) at least three electrodes (10) made of conductive fabric having conductive fiber and nonconductive fiber;
b) a transmitter converting unit (30) connected to the three electrodes (10) by at least three conductive yarn (20) while the transmitter converting unit (30) receives an input signal and converts it into a transmitted signal;
c) a wireless transmission module (40) for receiving the transmitted signal and producing a corresponding wireless signal; and
d) a transmitting antenna (50) receiving the signal and transmitting the wireless signal into a wireless receiver (60);
wherein,
outside the electrode (10) made of conductive fabric and the conductive yarn (20), there are
i) an electromagnetic interference shield layer (70),
ii) an isolation layer (80), and
ii) a conductive wire fixing layer (90) from outside in.

2. The wireless transmitted electrocardiogram monitoring device as claimed in claim 1, wherein surface resistivity of the electrode (10) made of conductive fabric (10) is less than 20 Ω/cm².

3. The wireless transmitted electrocardiogram monitoring device as claimed in claim 1, said device being adapted such that, in use, the conductive yarn (20) has no contact with the human body being detected.

4. The wireless transmitted electrocardiogram monitoring device as claimed in claim 1, said device being adapted such that, in use, at least two of the electrodes (10; 12; 14) are disposed on the fourth rib counting backwards of right and left chest respectively while one electrode (10; 16) is (located) arranged on back of a human body being detected and is contacted with the human body being detected.

5. The wireless transmitted electrocardiogram monitoring device as claimed in claim 1, wherein the wireless receiver (60) is connected to a remote terminal and wireless signals received by the wireless receiver (60) are transmitted to the remote terminal for being executed.

6. The wireless transmitted electrocardiogram monitoring device as claimed in claim 1, wherein the wireless receiver (60) is a personal digital assistant having a bluetooth module.

7. The wireless transmitted electrocardiogram monitoring device as claimed in claim 1, wherein the electromagnetic interference shield layer (70) is a shield for preventing electromagnetic waves woven by conductive yarn (72).

8. The wireless transmitted electrocardiogram monitoring device as claimed in claim 1, wherein the isolation layer (80) is woven by nonconductive yarn.

9. The wireless transmitted electrocardiogram monitoring device as claimed in claim 1, wherein the conductive wire fixing layer (90) is made by laminated fabric that is double layers structure for fixing and securing the conductive yarn (20).

## Patentansprüche

1. Überwachungsvorrichtung für drahtlos gesendetes Elektrokardiogramm, die umfasst:
a) wenigstens drei Elektroden (10), die aus einem leitenden Gewebe hergestellt sind, das leitende Fasern und nicht leitende Fasern besitzt;
b) eine Senderumsetzungseinheit (30), die mit den drei Elektroden (10) über wenigstens drei leitende Garne (20) verbunden ist, wobei die Senderumsetzungseinheit (30) ein Eingangssignal empfängt und es in ein gesendetes Signal umsetzt;
c) ein Drahtlossendemodul (40), um das gesendete Signal zu empfangen und um ein entsprechendes Drahtlossignal zu erzeugen; und
d) eine Sendeantenne (50), die das Signal empfängt und das Drahtlossignal an einen Drahtlosempfänger (60) sendet;
wobei
außerhalb der aus einem leitenden Gewebe hergestellten Elektrode (10) und des leitenden Garns (20) vorhanden sind:
i) eine Schicht (70) zur Abschirmung elektromagnetischer Störung,
ii) eine Isolierschicht (80) und
iii) eine Leiterdraht-Befestigungsschicht (90) von außen nach innen.

2. Überwachungsvorrichtung für drahtlos gesendetes Elektrokardiogramm nach Anspruch 1, wobei der spezifische Oberflächenwiderstand der aus leitendem Gewebe (10) hergestellten Elektrode (10) weniger als 20 Ω/cm² beträgt.

3. Überwachungsvorrichtung für drahtlos gesendetes Elektrokardiogramm nach Anspruch 1, wobei die Vorrichtung dazu ausgelegt ist, dass im Gebrauch das leitende Garn (20) keinen Kontakt mit dem detektierten menschlichen Körper hat.

4. Überwachungsvorrichtung für drahtlos gesendetes Elektrokardiogramm nach Anspruch 1, wobei die Vorrichtung dazu ausgelegt ist, dass im Gebrauch wenigstens zwei der Elektroden (10; 12; 14) auf der vierten Rippe bei Rückwärtszählung der rechten bzw. der linken Brust angeordnet sind, wobei (sich) eine Elektrode (10; 16) auf dem Rücken des detektierten menschlichen Körpers angeordnet ist (befindet) und mit dem detektierten menschlichen Körper in Kontakt ist.

5. Überwachungsvorrichtung für drahtlos gesendetes Elektrokardiogramm nach Anspruch 1, wobei der Drahtlosempfänger (60) mit einem entfernten Endgerät verbunden ist und Drahtlossignale, die von dem Drahtlosempfänger (60) empfangen werden, an das entfernte Endgerät gesendet werden, um ausgeführt zu werden.

6. Überwachungsvorrichtung für drahtlos gesendetes Elektrokardiogramm nach Anspruch 1, wobei der Drahtlosempfänger (60) ein persönlicher digitaler Assistent mit einem Bluetooth-Modul ist.

7. Überwachungsvorrichtung für drahtlos gesendetes Elektrokardiogramm nach Anspruch 1, wobei die Schicht (70) zur Abschirmung elektromagnetischer Störung eine Abschirmung ist, um elektromagnetische Wellen abzuweisen, die aus leitendem Garn (72) gewebt ist.

8. Überwachungsvorrichtung für drahtlos gesendetes Elektrokardiogramm nach Anspruch 1, wobei die Isolierschicht (80) aus nicht leitendem Garn gewebt ist.

9. Überwachungsvorrichtung für drahtlos gesendetes Elektrokardiogramm nach Anspruch 1, wobei die Leiterdraht-Befestigungsschicht (90) aus laminiertem Gewebe hergestellt ist, das eine Doppelschichtstruktur ist, um das leitende Garn (20) zu fixieren und zu befestigen.

## Revendications

1. Dispositif de surveillance d'électrocardiogramme émis sans fil comprenant :
a) au moins trois électrodes (10) réalisées dans un tissu conducteur qui présente des fibres conductrices et des fibres non conductrices ;
b) une unité de conversion d'émetteur (30) connectée aux trois électrodes (10) par au moins trois fils conducteurs (20) tandis que l'unité de conversion d'émetteur (30) reçoit un signal d'entrée et le convertit en un signal émis ;
c) un module d'émission sans fil (40) destiné à recevoir le signal émis et à produire un signal sans fil correspondant ; et
d) une antenne d'émission (50) destinée à recevoir le signal et à émettre le signal sans fil vers un récepteur sans fil (60) ;
dans lequel,
à l'extérieur de l'électrode (10) constituée d'un tissu conducteur et du fil conducteur (20), il y a :
i) une couche de protection vis-à-vis des interférences électromagnétiques (70) ;
ii) une couche isolante (80) ; et
iii) une couche de fixation des fils conducteurs (90) de l'extérieur vers l'intérieur.

2. Dispositif de surveillance d'électrocardiogramme émis sans fil selon la revendication 1, dans lequel la résistivité de la surface de l'électrode (10) constituée d'un tissu conducteur (10) est inférieure à 20 Ω/cm².

3. Dispositif de surveillance d'électrocardiogramme émis sans fil selon la revendication 1, dans lequel ledit dispositif est adapté de telle sorte que, en service, le fil conducteur (20) n'ait aucun contact avec le corps humain détecté.

4. Dispositif de surveillance d'électrocardiogramme émis sans fil selon la revendication 1, dans lequel ledit dispositif est adapté de telle sorte que, en service, au moins deux des électrodes (10 ; 12 ; 14) soient disposées sur la quatrième côte en comptant en arrière de la poitrine droite et gauche respectivement tandis qu'une électrode (10 ; 16) est (située) disposée sur le dos d'un corps humain détecté et est en contact avec le corps humain détecté.

5. Dispositif de surveillance d'électrocardiogramme émis sans fil selon la revendication 1, dans lequel le récepteur sans fil (60) est connecté à un terminal distant et les signaux sans fil reçus par le récepteur sans fil (60) sont émis vers le terminal distant pour être exécutés.

6. Dispositif de surveillance d'électrocardiogramme émis sans fil selon la revendication 1, dans lequel le récepteur sans fil (60) est un assistant numérique personnel qui dispose d'un module Bluetooth.

7. Dispositif de surveillance d'électrocardiogramme émis sans fil selon la revendication 1, dans lequel la couche de protection vis-à-vis des interférences électromagnétiques (70) est un blindage destiné à empêcher les ondes électromagnétiques générées par le fil conducteur (72).

8. Dispositif de surveillance d'électrocardiogramme émis sans fil selon la revendication 1, dans lequel la couche isolante (80) est tissée dans un fil non conducteur.

9. Dispositif de surveillance d'électrocardiogramme émis sans fil selon la revendication 1, dans lequel la couche de fixation du fil conducteur (90) est réalisée dans un tissu stratifié qui présente une structure à deux couches destinée à fixer le fil conducteur (20).
